# EUROPEAN PATENT SPECIFICATION

(11) **EP 4 078 618 B1**
(45) Date of publication and mention of the grant of the patent: **30.09.2026**
(21) Application number: 20845715.0
(22) Date of filing: 18.12.2020
(51) Int. Cl.: G16H 50/20, A61B 5/08

(54) **SYSTEMS AND METHODS FOR COPD MONITORING**
SYSTEME UND VERFAHREN ZUR ÜBERWACHUNG VON COPD
SYSTÈMES ET PROCÉDÉS DE SURVEILLANCE DE BRONCHOPNEUMOPATHIE CHRONIQUE OBSTRUCTIVE (COPD)

(30) Priority: 20.12.2019 US 201962951383 P
(43) Date of publication of application: 26.10.2022
(73) Proprietor: ResMed Inc., San Diego CA 92123 (US)
(72) Inventor: KENNEDY, Colin, Bradley, San Diego, California 92123 (US)
(74) Representative: Mathys & Squire
(86) International application number: PCT/US2020/066070
(87) International publication number: WO 2021/127478

(56) References cited:
- WO-A1-2019/229543
- KR-A- 20160 133 200
- US-A1- 2011 124 589
- US-A1- 2016 004 834
- US-A1- 2019 383 830
- TAHER FATMA ET AL: "Comparison of Hopfield Neural Network and mean shift algorithm in segmenting sputum color images for lung Cancer Diagnosis", 2013 IEEE 20TH INTERNATIONAL CONFERENCE ON ELECTRONICS, CIRCUITS, AND SYSTEMS (ICECS), IEEE, 8 December 2013 (2013-12-08), pages 649 - 652, XP032595323, DOI: 10.1109/ICECS.2013.6815498
- RIA LESTARI MOEDOMO ET AL: "The breath sound analysis for diseases diagnosis and stress measurement", SYSTEM ENGINEERING AND TECHNOLOGY (ICSET), 2012 INTERNATIONAL CONFERENCE ON, IEEE, 11 September 2012 (2012-09-11), pages 1 - 6, XP032264327, ISBN: 978-1-4673-2375-8, DOI: 10.1109/ICSENGT.2012.6339358

## Description

### CROSS-REFERENCE TO RELATED APPLICATION

This application claims the benefit of, and priority to, U.S. Provisional Patent Application No. 62/951,383, filed December 20, 2019.

### BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders, namely systems and methods for monitoring COPD. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

### 1.2.2 Therapies

Various therapies, such as Continuous Positive Airway Pressure (CPAP) therapy, Non-invasive ventilation (NIV) and Invasive ventilation (IV) have been used to treat one or more of the above respiratory disorders.

### 1.2.3 Treatment Systems

These respiratory therapies may be provided by a therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include CPAP devices and ventilators. Examples of RPT devices include a CPAP device and a ventilator.

### 1.2.3.3 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 1.2.3.4 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

### 1.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

US 2016/004834 A1 relates to a method and system for managing a chronic medical condition, such as chronic pulmonary obstructive disorder. The method includes generating a baseline score for a patient; recording an exacerbation severity for a plurality of symptoms; weighting the recorded exacerbation severities; determining an exacerbation score relative to the baseline score based on at least one of the recorded or weighted exacerbation severities; assigning the exacerbation score to a category; publishing the exacerbation score and assigned category for review by a physician or medical professional; and transmitting a treatment plan to the patient, the treatment plan being prescribed by a physician and based at least in part on the exacerbation score and/or assigned category. The system includes a computer that allows the patient to perform the steps of the method and a triage center for evaluating the published exacerbation scores and prescribing and communicating a treatment plan for the patient.

### BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology is to provide monitoring of patients with COPD. Systems and methods for monitoring COPD are defined by the claims.

An aspect of certain forms of the present technology is to provide methods and/or apparatus that improve the compliance of patients with respiratory therapy.

An aspect of certain forms of the present technology is to provide monitoring of patients with COPD, including the progression of the disease through various stages and monitoring for certain

One form of the present technology comprises image processing techniques that identifies a COPD disease progression trajectory.

Also disclosed herein is the technology to alert caregivers if it is determined a patient will experience a COPD exacerbation event within an hour, a few minutes, or a day, or other time range.

Another aspect of one form of the present technology is the use of cameras and/or infrared sensors to sense a color, shape, volume, and surface temperature and other visual feature changes of sputum produced by a patient with COPD to determine the trajectory of the COPD.

An aspect of certain forms of the present technology is a medical device that is easy to use, e.g. by a person who does not have medical training, by a person who has limited dexterity, vision or by a person with limited experience in using this type of medical device.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, namely COPD.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### BRIEF DESCRIPTION OF THE DRAWINGS

The present technology is illustrated by way of example, and not by way of limitation, in the figures of the accompanying drawings, in which like reference numerals refer to similar elements including:

### 1.3 TREATMENT SYSTEMS

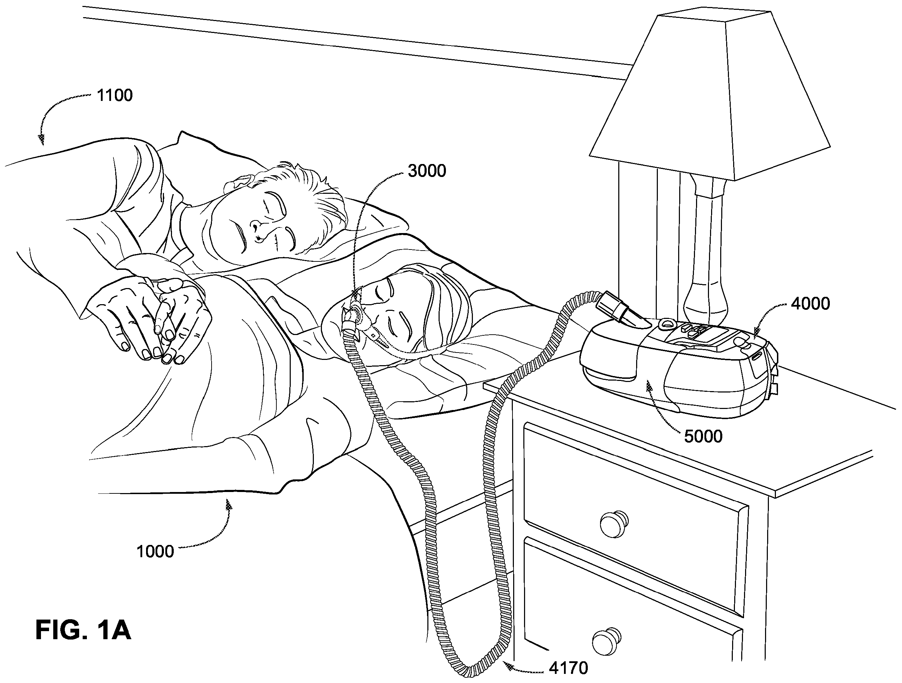

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

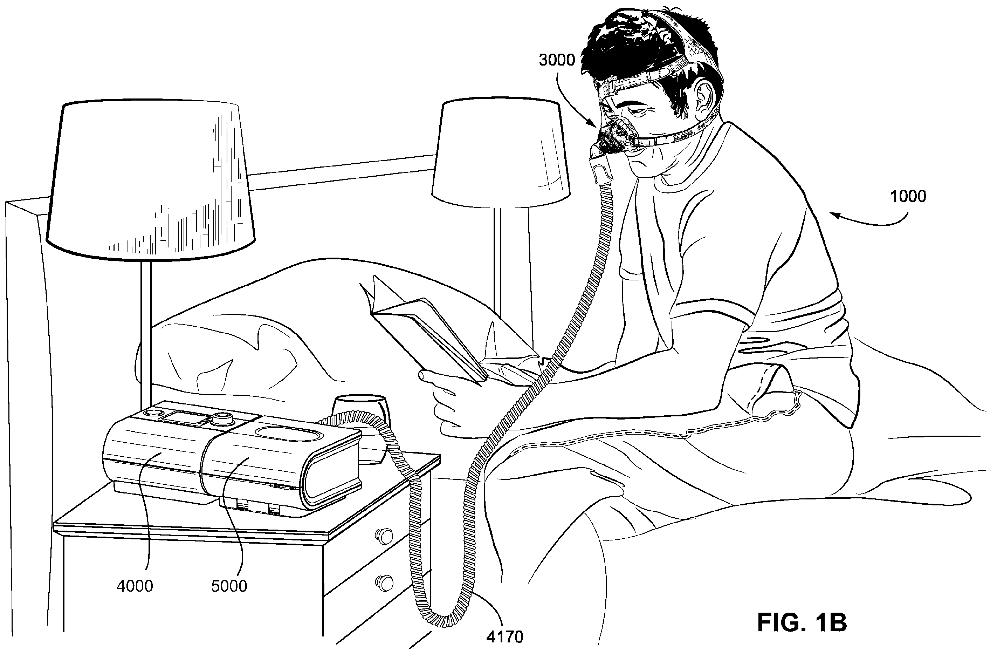

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

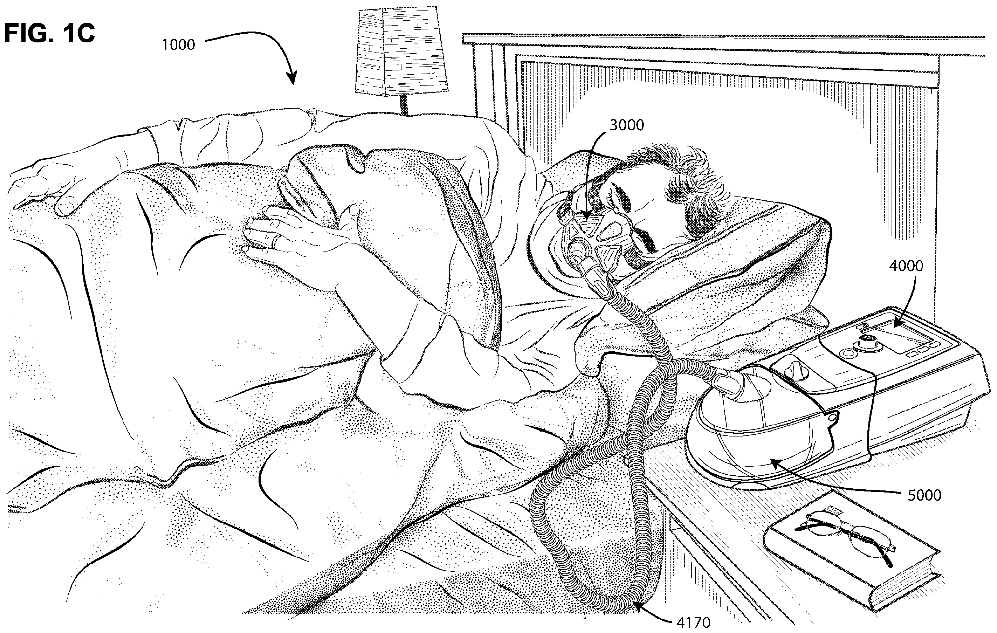

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 1.4 RESPIRATORY SYSTEM AND FACIAL ANATOMY

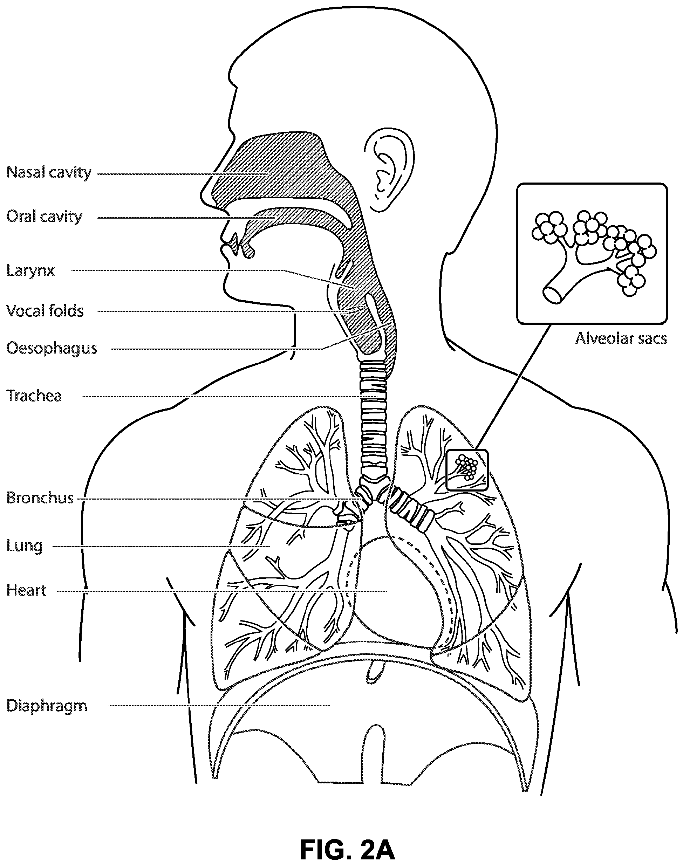

Fig. 2A shows an overview of a human respiratory system including the nasal and oral cavities, the larynx, vocal folds, oesophagus, trachea, bronchus, lung, alveolar sacs, heart and diaphragm.

### 1.5 PATIENT INTERFACE

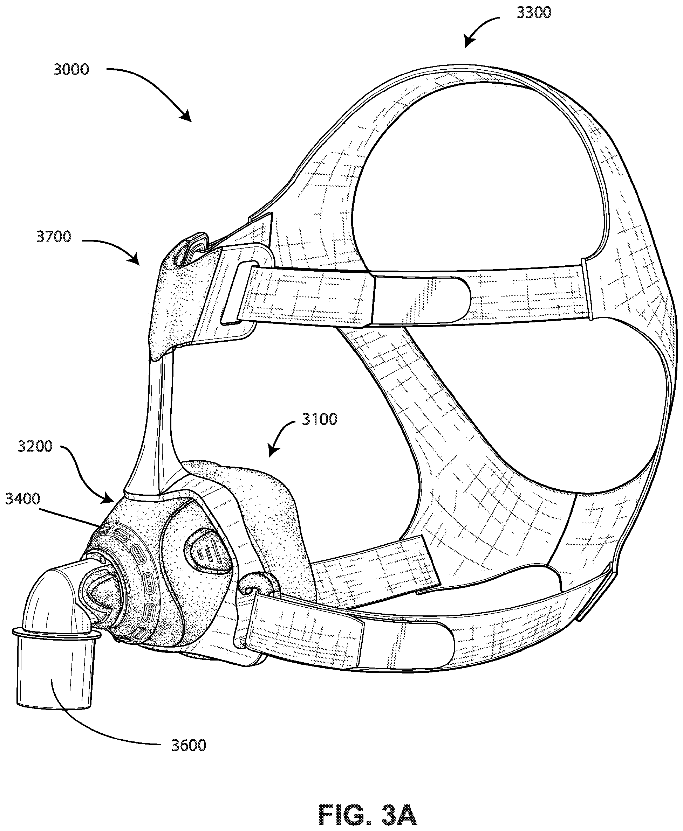

Fig. 3A shows a patient interface in the form of a nasal mask in accordance with one form of the present technology.

### 1.6 RPT DEVICE

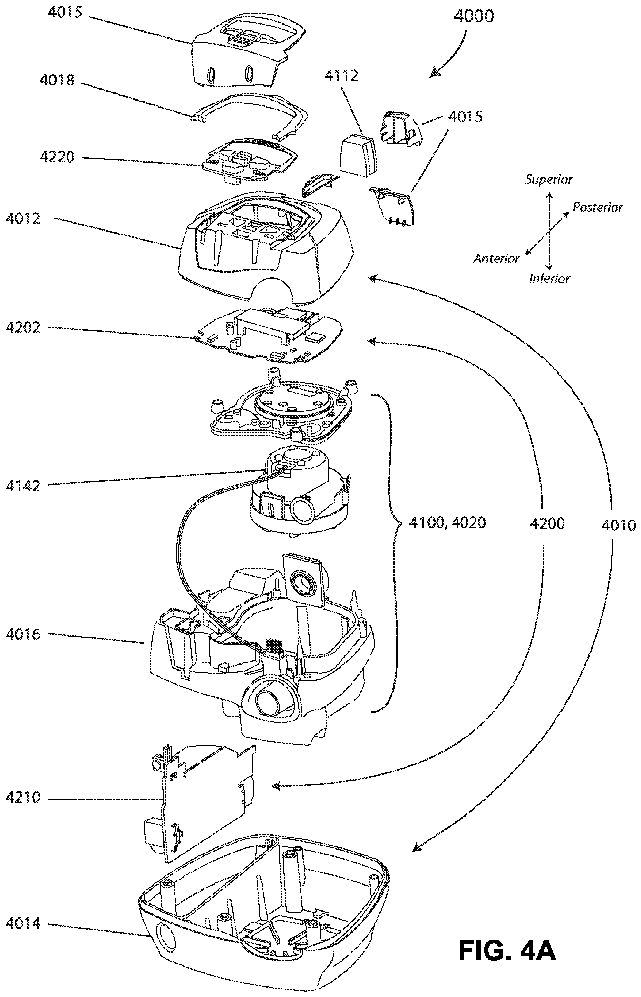

Fig. 4A shows an RPT device in accordance with one form of the present technology.

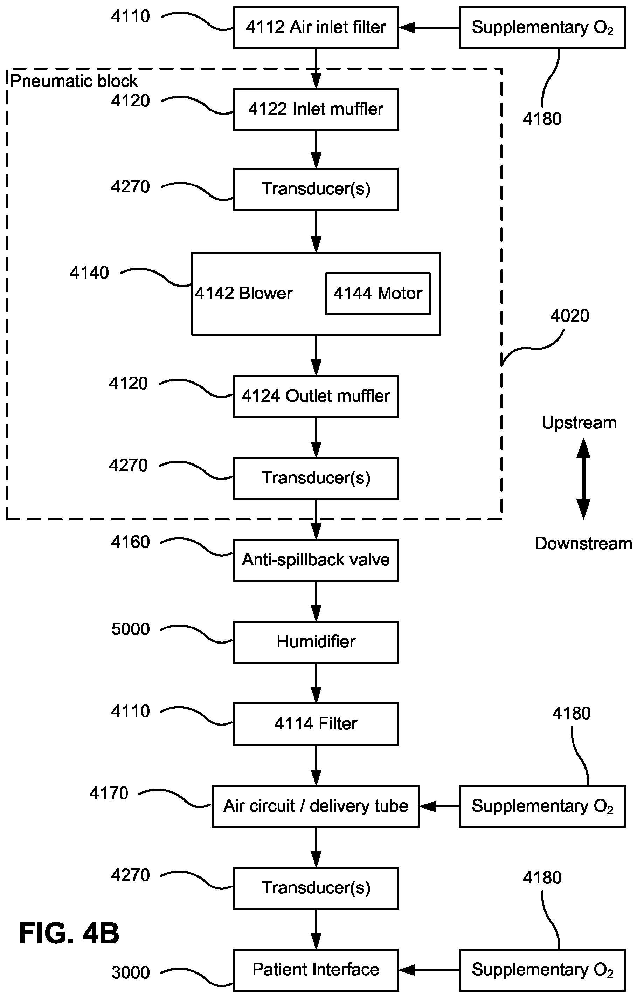

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 1.7 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 1.8 BREATHING WAVEFORMS

Fig. 6A shows a model typical breath waveform of a person while sleeping.

### 1.9 SCREENING, DIAGNOSIS AND MONITORING SYSTEMS

Fig. 7 shows an overview of an example system for monitoring a patient with COPD.
Fig. 8 shows a flow chart of an example process for monitoring a patient with COPD.
Fig. 9 shows a flow chart of an example process for monitoring a patient with COPD.
Fig. 10 shows a flow chart of an example process for monitoring a patient with COPD.

### DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 1.10 THERAPY

In one form, the present technology comprises a method for treating a respiratory disorder comprising the step of applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 1.11 TREATMENT SYSTEMS

In one form, the present technology comprises an apparatus or device for treating a respiratory disorder. The apparatus or device may comprise an RPT device 4000 for supplying pressurised air to the patient 1000 via an air circuit 4170 to a patient interface 3000 or 3800.

### 1.12 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.

An unsealed patient interface 3800, in the form of a nasal cannula, includes nasal prongs 3810a, 3810b which can deliver air to respective nares of the patient 1000. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 lead to an RT device that generates the flow of air at high flow rates. The "vent" at the unsealed patient interface 3800, through which excess airflow escapes to ambient, is the passage between the end of the prongs 3810a and 3810b of the cannula 3800 via the patient's nares to atmosphere.

### 1.13 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.

### 1.14 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

### 1.14.1 Humidifier overview

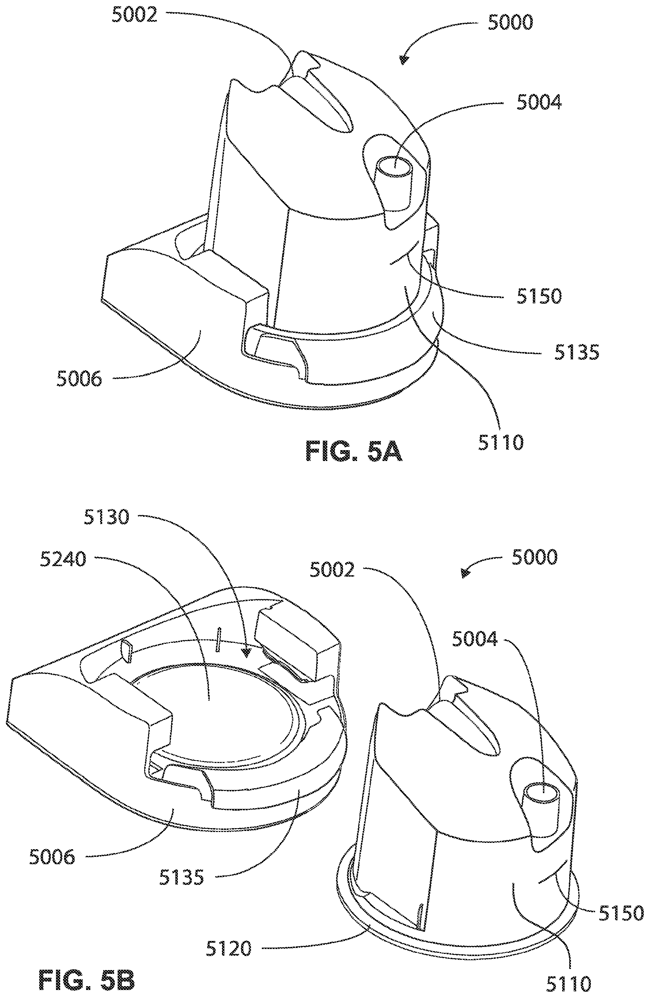

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 1.15 BREATHING WAVEFORMS

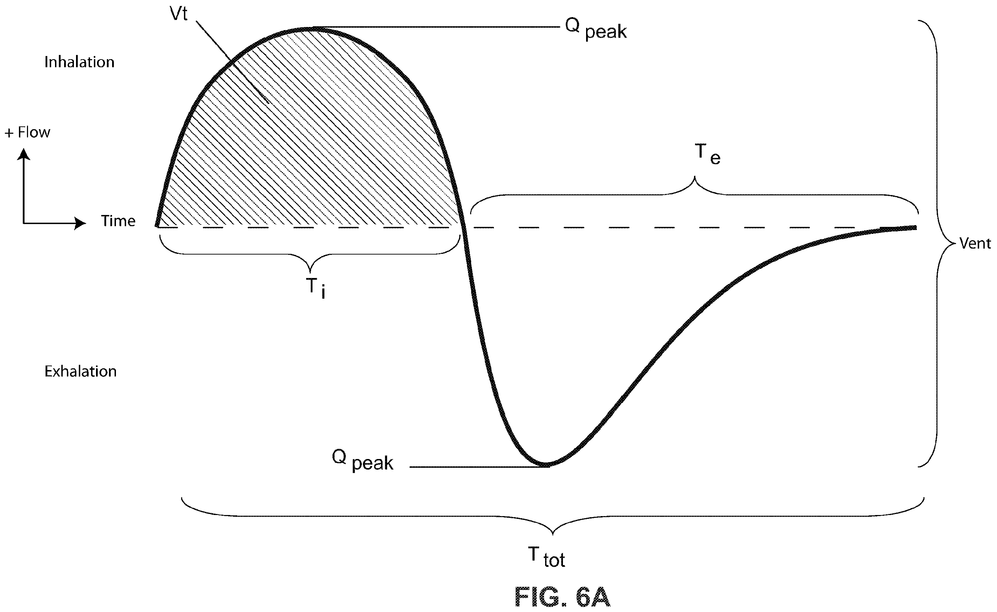

Fig. 6A shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 1.15.1 COPD Screening, Diagnosis, and Monitoring

Patients with chronic obstructive pulmonary disease ("COPD") have an unpredictable trajectory which eventually results in death. Research has found that by identifying "transitions points" it would allow identification of those patients who may benefits from a palliative approach to their care, or referral to a specialist palliative care service, or preventive care to avoid or minimize an exacerbation event.

Particularly, patients with COPD follow a chronic disease trajectory, with longer periods of declining health with interspersed exacerbations event that may cause death. The course of events is highly unpredictable with currently available methods. The milestones may include: (1) loss of recreation, (2) home environment (downsizing or requiring residential care), (3) episodes of acute care, (4) long-term oxygen treatment, (5) dyspnoea episodes, (6) assistance with self-care required, and (7) others. These milestones may occur in any order. In some cases, exacerbation events will increase in frequency before death.

Patients with severe COPD require a complex set of caregivers and interventions that may need to be called upon rapidly. Accordingly, a need exists to determine and predict when and what type of assistance may be needed. For instance, non-invasive ventilation has shown to be one of the few viable treatments for COPD. Furthermore, additional practical assistance that is needed may be unpredictable. This may include: (1) primary care, (2) palliative care, (3) ambulance services, (4) non-invasive ventilation, (5) assistance with self-care activities, and others.

Accordingly, systems and methods have been developed to monitor and predict the course of COPD in a patient, and predict exacerbation events and other care needs. This includes systems and methods that may be performed by the patient in the home in some cases, and some that utilized sensors available on mobile devices. Thus, patients may be able to at least partially manage COPD at home without the intervention of caregivers and reduce the costs of admission to hospitals. Furthermore, the systems and methods may determine when an exacerbation event is likely developing to immediately alert the patient and caregivers, so that appropriate care can be delivered to mitigate the event.

For instance, with daily monitoring of various factors disclosed herein, the disclosed systems and methods may be able to predict exacerbation events, or transition periods requiring much different or increased care with greater accuracy. Additionally, this may be performed without requiring expensive patient provider visits or services to test clinical signs of the systems (e.g. respiratory tests).

### 1.16 COPD MONITORING SYSTEMS

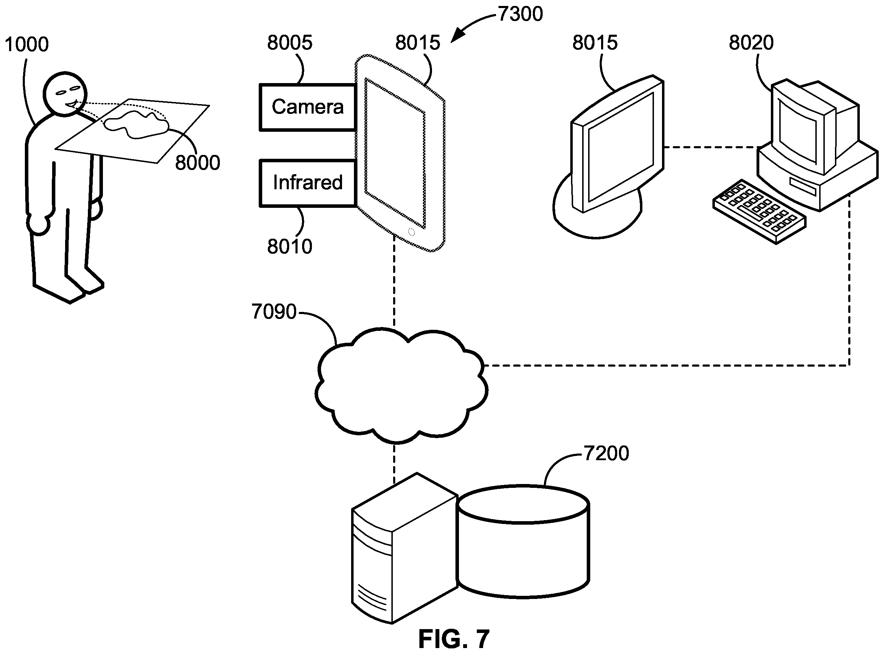

FIG. 7 illustrates an example of a COPD monitoring system according to the present disclosure. The system may include a patient 1000 that has coughed up a sputum 8000 and a mobile device 7300 with a camera 8005 and microphone 8010. The sputum 8000 may be phlegm, mucous, or other liquid substance coughed up by a patient 8000 during a coughing episode that may be visually captured by the camera 8005 to output image data representing the sputum and the sounds of coughing during the sputum event may be recorded by the microphone 8010. The sputum 8000 may be different sizes, and may be at any stage of the COPD process.

The camera 8005 and microphone 8010 may be incorporated into a mobile device 7300 or may be external and separate. In some examples, the camera 8005 may be utilized to capture images of the sputum 8000 and the microphone 8010 may be utilized to capture sounds of a patient coughing up sputum. The camera 8005 may be a RGB, black and white camera, and may include multiple cameras to detect images from different points of view.

The mobile device 7300 may be a cell phone, tablet, or other suitable computing device. The mobile device 7300 may be connected to a wired or wireless network 7090 and may transmit image data and other data related to the wound 8000 over the network 7090. The mobile device 7300 may include a display and interactive user interface (e.g. touch screen).

The network 7090 may be connected to other computing device 8020 and displays 8015, and may also be connected to a server 7100 and database 7200. In some examples, the processing of image data and other sensor data may take place on the mobile device 7300, on the computing device 8020 or on the remote server 7100. In some examples, certain portions of the processing (e.g. computer vision) that require heavier CPU load may take place on the server 7100. In some examples, scaled down versions of machine learning algorithms may run locally on the mobile device 7300.

### 1.17 COPD MONITORING METHODS

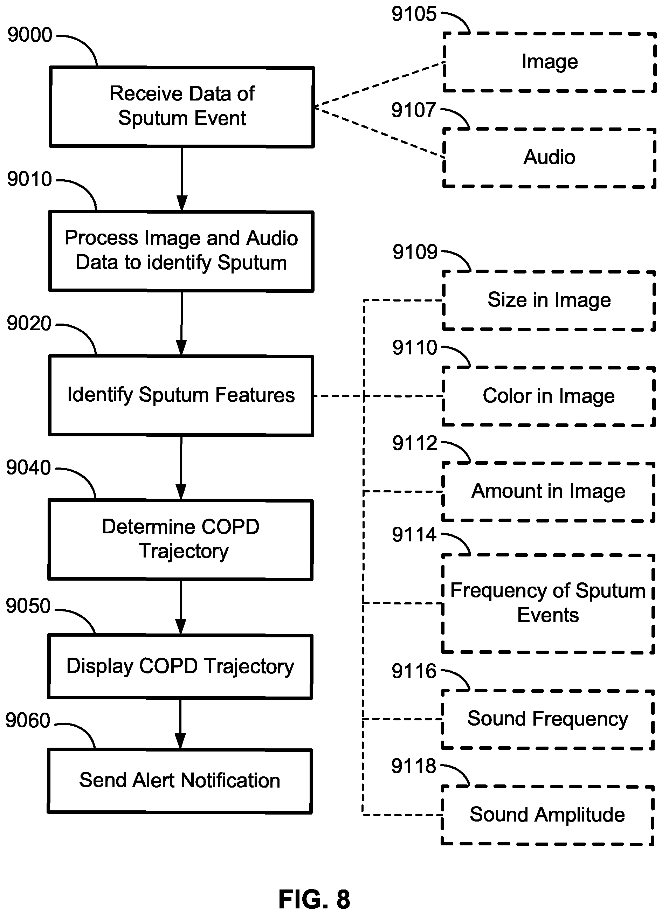

FIG. 8 is a flow chart illustrating an example process for monitoring, diagnosing and predicting a COPD trajectory. In some examples, these steps are performed by a mobile device 7300, computing device 7020, or on a remote server 7100. Various steps may be performed on separate computing devices and control systems or the steps may all be performed by a single processor.

First, the system may receive data after a sputum event 9000. This may be an event where the patient 1000 coughs up sputum 8000. In some examples, the system may continually monitor for coughing sounds and initiate recording of audio data 9107 with the microphone 8010 if a certain amplitude, frequency, and/or duration of coughing is detected by a passive audio monitoring of audio data 9107 output from microphone 8010.

In some examples, the system may display a question on an interface of a mobile device as to whether a sputum event occurred. In that case, the audio data 9107 may be saved, time stamped and labelled a sputum event, and the system may then request the patient 1000 take a picture of the sputum 8000 with the camera 8005 to output image data 9105 of the sputum.

Next, the system may process the image and audio data to identify sputum 9010 in the image. This may include various image processing and computer vision algorithms known in the art for identifying boundaries of sputum in the image including color and size based algorithms that identify typical sputum colors (e.g. white, yellow, clearish, brown, or red for blood).

Next, the system may identify sputum features within the sputum boundaries 9020. This may include size 9109, color 9110, amount 9112 (e.g. volume), frequency of sputum events 9114, frequency of coughing sound 9116, and amplitude of coughing sound 9118. The size 9109, amount 9112 (e.g. volume) and other features may be determined by surface area of the sputum, and/or by depth sensors (determining varying depths of the sputum to get a maximum and minimum height, for instance to estimate volume). In some examples, a distance of the phone to the sputum may be required to get a determination of size 9109 or amount 9112, or in some cases a user may be instructed to place a reference object in the background.

The features based on sound data may various sounds features of the coughing or breathing, including amplitude 9118, frequency 9116, length of coughing episode, averages of these features, trends of these features and others. For instance, noisy or increased breathing rates and/or coughing can be a sign of an oncoming exacerbation event.

The system may also identify the color 9110 or various other optical features of the sputum 8000. These features may primarily be determined from the image data output from the camera 8005. For instance, various colors 9110 may indicate infections or various stages of COPD including clear, white, green, grey, yellow, brownish and red. Additionally, various colors 9110 may indicate an event is likely to happen soon and an alert can be sent out.

Accordingly, once the computer vision algorithms have identified the relevant sputum 8000 features, the system may determine a COPD trajectory 9040. The COPD trajectory may be determined based on a variety of factors which may include the frequency of coughing and amount of sputum 8000. In some examples, whether or not sputum is associated with a cough and the frequency it is (whether or not a patient 1000 coughs up sputum) may be utilized to determine the trajectory and or stage. For instance, progression along the stages is associated with an increase in coughing and sputum production.

The COPD trajectory may be displayed 9060 and may include a variety of outputs, including the following:
- COPD stage;
- time remaining at stage;
- progress versus average based on the timeline;
- quantitative measures of COPD progress;
- resources that are helpful for a given stage; and
- others.

Then, the system may display the trajectory 9040 on the mobile device 7300 or other computing devices and display 8015.

The system could also analyse the COPD trajectory to determine if an alert is necessary 9050. For instance, the system may also send a notification or alert 9060 if the sputum is a certain color, for instance indicating it includes blood.

In another example, an alert may be sent 9060 if an exacerbation event is predicted to occur in or short time window, for instance a few minutes, an hour, the same day or the same week. Depending on the predicted time window for the exacerbation event, different caregivers may be notified depending on the urgency (within a few minutes emergency personnel may be notified). Signs of exacerbation events include changes in breathing sounds, irregular breathing, coughing that is worsening in severity (e.g. amplitude or frequency of episodes) or other signs.

The alert may issue from the mobile device 7300 or other computing device 8020. Thus, the system could send a variety of notifications including:
- an SMS message to the mobile device 7300;
- call the mobile device 7300;
- alert the care giver;
- issue an alert with the application; or
- others.

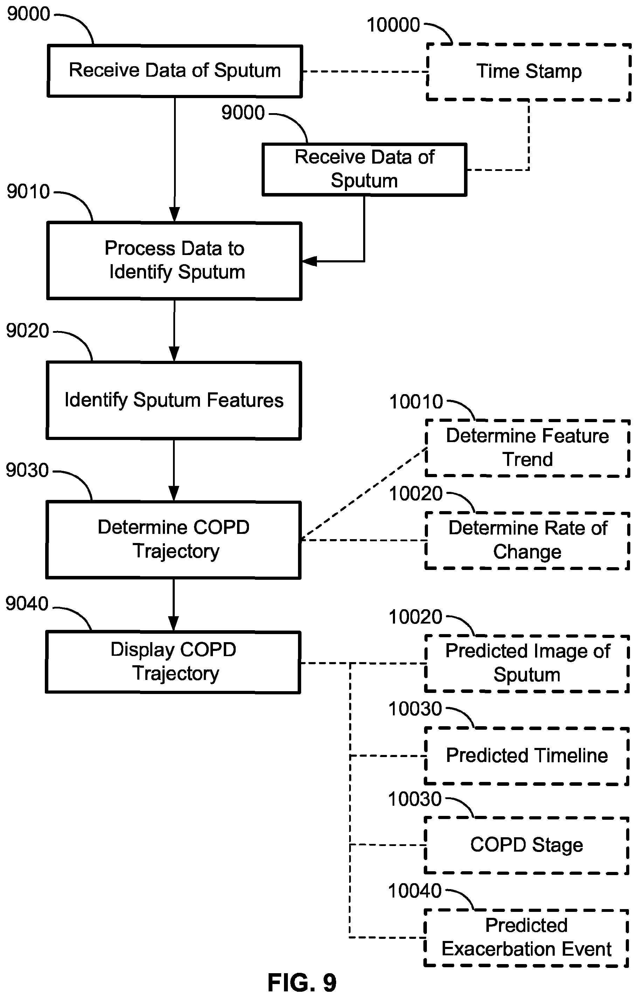

FIG. 9 illustrates another example of a monitoring process that includes processing images from more than one point in time. The system receives a first image of the sputum 9000 with a first time stamp 10000 and receives a second image of the sputum 9000 with a second time stamp 10000. Then, the system determines the time elapsed between the two images and may determine estimated changes between the sputum in the images (e.g. increase in size, change in color, amount of certain colors). Accordingly, this can be utilized to determine or estimate the trajectory of the wound 9000 based on its improvement rate.

For instance, as disclosed herein, the sputum boundaries could be identified 9010 and then the sputum features within those boundaries could be identified 9020. This could be performed for both images. In some examples, the system may first determine a likelihood the sputum is the same sputum, based on the time elapsed and the similarity of the features.

Next, when the COPD trajectory is determined 9030, the system may determine a feature trend 10010 and/or determine a rate of change of the features 10020. For instance, the system could determine an increase in amplitude/amount or frequency of coughing, breathing, or sputum, and determine a rate of change of those features. In some example, the system may determine how frequently sputum accompanies coughing for instance.

The trends 10010 could then be utilized to extrapolate a COPD trajectory based on the trends 10010 and the starting date or point or determine a COPD stage and/or progression through a COPD stage. Accordingly, the COPD trajectory could then be displayed 9040. and/or a predicted timeline to reach the next stage 10030.

In other examples, the system may utilize two, three, four, five images of sputum 9000, and associated audio data of breathing and/or coughing to determine a trend and trajectory 9030.

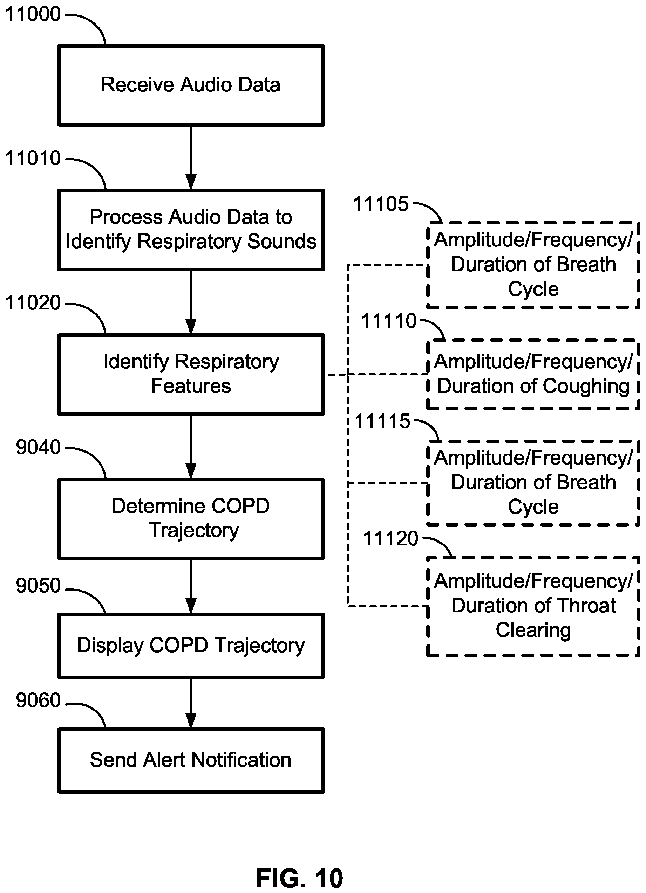

FIG. 10 illustrates another example of the monitoring process using audio data. For instance, in some examples, the system may receive audio data 11000, and process the audio data to identify respiratory sounds 11010. This may require continually processing audio data output from a microphone (for instance on a smartphone of a user) to determine whether portions of the audio data 11000 may be relevant or useful for further processing.

For instance, the breathing could be periodically monitored or the audio could be periodically monitored and temporarily stored until threshold conditions are met. This may include an audio signature that indicates coughing including a certain amplitude and/or frequency. Accordingly, the system could process the audio data to identify audio data representing coughing episodes and discarding the other audio data.

In other examples, the system may not be able to detect a user breathing unless the microphone is held up to the user's mouth. Accordingly, on a regular interval (e.g. once a day, twice a day, twice a week, once a week), a user interface may notify a patient 1000 to hold a smartphone up to their mouth or other device so that audio data representing the patient breathing maybe recorded.

Accordingly, once audio data 11000 of interest is recorded and captured (and/or identified in the case of continuous monitoring and coughing) the audio data 11000 may be processed to identify or separate the specific respiratory sounds 11010. This may include certain frequency filters as coughing and breathing have a particular frequency window.

Then, the processed data may be further processed to identify respirator features. This may include the amplitude/frequency/duration of the breath cycle 11105, the amplitude/frequency/duration of coughing 11110, the amplitude/frequency/duration of wheezing 11115, and the amplitude/frequency/duration of throat clearing 11120. All of these features may be relevant to the progression of COPD through the various stages and these features may then be processed (or subsets or combinations of features) to determine a COPD trajectory 9040, display the trajectory 9050 and an alert 9060 if applicable as disclosed in further detail herein.

### 1.18 COPD MONITORING AND SLEEP MONITORING

In some examples, the system may also input sleep quality metrics that are output by a respiratory therapy device 4000 to modify the predicted COPD trajectory 9030. This may include sleep quality (e.g. sleep score) or may include total sleep time per night. In some examples, the system could train a model of healing based on user data from various images and sleep data, to determine how much an improvement in sleep will slow down the progression of COPD.

In other examples, the predicted time to the next COPD stage could be automatically modified based on the patient's 1000 sleep score. Additionally, the system could determine a target amount of sleep for a patient 1000 to significantly slow down the progression of the COPD predicted timeline 10030.

### 1.19 DETECTION OF COMORBID CONDITIONS

In some examples, the predicted trajectory may take into account comorbid conditions that may be exacerbating the COPD, and therefore additional interventions may not be required or may indicate the trajectory is stable despite exacerbations. This may include the presence of respiratory infections such as bronchitis, pneumonia, tuberculosis, or others. In some examples, the system will be trained to identify comorbidities, or the user may input comorbidities after diagnosis by a health care provider.

### 1.20 GLOSSARY

For the purposes of the present technology disclosure, in certain forms of the present technology, one or more of the following definitions may apply. In other forms of the present technology, alternative definitions may apply.

### 1.20.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol *Q.* 'Flow rate' is sometimes shortened to simply 'flow' or 'airflow'.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Patient:* A person, whether or not they are suffering from a respiratory condition.

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 1.20.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker.

*Polycarbonate:* a thermoplastic polymer of Bisphenol-A Carbonate.

### 1.21 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the claimed invention, which is defined by the following claims.

## Claims

1. A system for monitoring a COPD trajectory, the system comprising:
a display
a camera configured to output image data;
a memory containing machine readable medium comprising machine executable code having stored thereon instructions;
a control system coupled to the memory comprising one or more processors, the control system configured to execute the machine executable code to cause the control system to:
receive a first image output from the camera with a first time stamp and a second image output from the camera with a second time stamp;
compare the first time stamp and the second time stamp to determine a time period between the first image and the second image
process the first image and the second image to identify a first portion of pixels within the first image and a second portion of pixels within the second image that represent a sputum;
process the first portion to identify a first set of sputum features;
process the second portion to identify a second set of sputum features;
determine a COPD trajectory based on the first set of sputum features, the second set of sputum features, and the time period; and
display the COPD trajectory on the display.

2. The system of claim 1, wherein the first set of sputum features comprise at least one of: color, quantity, volume, and blood.

3. The system of claim 2, wherein quantity comprises surface area of sputum in an image.

4. The system of any one of claims 1 to 3, wherein a healing trajectory comprises a COPD stage or a predicted exacerbation event.

5. The system of claim 4, wherein the control system is further configured to process the first image to output a predicted image of the sputum after a first time interval.

6. The system of claim 5, wherein processing the first image comprises altering a color of a subset of the portion of the pixels.

7. The system of claim 5, wherein determining the healing trajectory further comprises receiving a first input from a user interface.

8. The system of any one of claims 1 to 7, wherein determining the healing trajectory comprises determining whether the patient is close to a milestone of a COPD trajectory.

9. The system of claim 8, wherein the control system is further configured to display a stock image associated with the milestone.

10. The system of any one of claims 1 to 9, wherein determining the COPD trajectory is based on a rate of change of the first set of sputum features and the second set of sputum features.

11. The system of any one of claims 1 to 9, wherein determining the COPD trajectory further comprises receiving a first input from a user interface.

12. A method for monitoring COPD, the method comprising:
receiving, at a control system comprising one or more processors, a first image output from a camera with a first time stamp and a second image output from the camera with a second time stamp;
comparing the first and second time stamps to determine a time period between the first and second images;
processing the first and second images to identify a first portion of the first image and a second portion of the second image that represent a sputum;
processing the first portion to identify a first set of sputum features and processing the second portion to identify a second set of sputum features;
processing the first and second set of sputum features and the time period to determine a COPD trajectory; and
displaying the COPD trajectory on the display.

## Patentansprüche

1. System zur Überwachung eines COPD-Verlaufs, wobei das System Folgendes umfasst:
eine Anzeige
eine Kamera, die so konfiguriert ist, dass sie Bilddaten ausgibt;
einen Speicher, der ein maschinenlesbares Medium enthält, das maschinenausführbaren Code umfasst, der darauf gespeicherte Anweisungen aufweist;
ein mit dem Speicher gekoppeltes Steuerungssystem, das einen oder mehrere Prozessoren umfasst, wobei das Steuerungssystem so konfiguriert ist, dass es den maschinenlesbaren Code ausführt, um zu bewirken, dass das Steuerungssystem:
ein erstes von der Kamera ausgegebenes Bild mit einem ersten Zeitstempel und ein zweites von der Kamera ausgegebenes Bild mit einem zweiten Zeitstempel empfängt;
den ersten Zeitstempel und den zweiten Zeitstempel vergleicht, um einen Zeitraum zwischen dem ersten Bild und dem zweiten Bild zu bestimmen;
das erste Bild und das zweite Bild verarbeitet, um einen ersten Abschnitt von Pixeln im ersten Bild und einen zweiten Abschnitt von Pixeln im zweiten Bild zu identifizieren, die Sputum darstellen;
den ersten Abschnitt verarbeitet, um einen ersten Satz von Sputummerkmalen zu identifizieren;
den zweiten Abschnitt verarbeitet, um einen zweiten Satz von Sputummerkmalen zu identifizieren;
einen COPD-Verlauf basierend auf dem ersten Satz von Sputummerkmalen, dem zweiten Satz von Sputummerkmalen und dem Zeitraum bestimmt; und
den COPD-Verlauf auf der Anzeige anzeigt.

2. System nach Anspruch 1, wobei der erste Satz von Sputummerkmalen mindestens eines umfasst von: Farbe, Menge, Volumen und Blut.

3. System nach Anspruch 2, wobei die Menge Oberflächenfläche des Sputums in einem Bild umfasst.

4. System nach einem der Ansprüche 1 bis 3, wobei ein Heilungsverlauf ein COPD-Stadium oder ein vorhergesagtes Exazerbationsereignis umfasst.

5. System nach Anspruch 4, wobei das Steuerungssystem weiter so konfiguriert ist, dass es das erste Bild verarbeitet, um nach einem ersten Zeitintervall ein vorhergesagtes Bild des Sputums auszugeben.

6. System nach Anspruch 5, wobei Verarbeiten des ersten Bildes Ändern einer Farbe eines Teilsatzes der Pixel umfasst.

7. System nach Anspruch 5, wobei Bestimmen des Heilungsverlaufs weiter Empfangen einer ersten Eingabe von einer Benutzerschnittstelle umfasst.

8. System nach einem der Ansprüche 1 bis 7, wobei Bestimmen des Heilungsverlaufs Bestimmen umfasst, ob sich der Patient in der Nähe eines Meilensteins eines COPD-Verlaufs befindet.

9. System nach Anspruch 8, wobei das Steuerungssystem weiter so konfiguriert ist, dass es ein dem Meilenstein zugeordnetes Archivbild anzeigt.

10. System nach einem der Ansprüche 1 bis 9, wobei Bestimmen des COPD-Verlaufs auf einer Änderungsrate des ersten Satzes von Sputummerkmalen und des zweiten Satzes von Sputummerkmalen basiert.

11. System nach einem der Ansprüche 1 bis 9, wobei Bestimmen des COPD-Verlaufs weiter Empfangen einer ersten Eingabe von einer Benutzerschnittstelle umfasst.

12. Verfahren zur Überwachung von COPD, wobei das Verfahren Folgendes umfasst:
Empfangen an einem Steuerungssystem, das einen oder mehrere Prozessoren umfasst, eines ersten von einer Kamera ausgegebenen Bildes mit einem ersten Zeitstempel und eines zweiten von der Kamera ausgegebenen Bildes mit einem zweiten Zeitstempel;
Vergleichen des ersten und des zweiten Zeitstempels, um einen Zeitraum zwischen dem ersten und dem zweiten Bild zu bestimmen;
Verarbeiten des ersten und zweiten Bildes, um einen ersten Abschnitt des ersten Bildes und einen zweiten Abschnitt des zweiten Bildes zu identifizieren, die Sputum darstellen;
Verarbeiten des ersten Abschnitts, um einen ersten Satz von Sputummerkmalen zu identifizieren und Verarbeiten des zweiten Abschnitts, um einen zweiten Satz von Sputummerkmalen zu identifizieren;
Verarbeiten des ersten und zweiten Satzes von Sputummerkmalen und des Zeitraums, um einen COPD-Verlauf zu bestimmen; und
Anzeigen des COPD-Verlaufs auf der Anzeige.

## Revendications

1. Système de surveillance d'une évolution de BPCO, le système comprenant :
un écran ;
une caméra configurée pour produire des données d'image ;
une mémoire contenant un support lisible par machine comprenant un code exécutable par machine sur lequel sont stockées des instructions ;
un système de commande couplé à la mémoire comprenant un ou plusieurs processeurs, le système de commande étant configuré pour exécuter le code exécutable par machine afin d'amener le système de commande à :
recevoir une première image produite par la caméra avec un premier horodatage et une seconde image produite par la caméra avec un second horodatage ;
comparer le premier horodatage et le second horodatage pour déterminer une période de temps entre la première image et la seconde image ;
traiter la première image et la seconde image pour identifier une première partie de pixels dans la première image et une seconde partie de pixels dans la seconde image qui représentent un crachat ;
traiter la première partie pour identifier un premier ensemble de caractéristiques de crachat ;
traiter la seconde partie pour identifier un second ensemble de caractéristiques de crachat ;
déterminer une évolution de BPCO sur la base du premier ensemble de caractéristiques de crachat, du second ensemble de caractéristiques de crachat et de la période de temps ; et
afficher l'évolution de BPCO sur l'écran.

2. Système selon la revendication 1, dans lequel le premier ensemble de caractéristiques de crachat comprend au moins un parmi : une couleur, une quantité, un volume et du sang.

3. Système selon la revendication 2, dans lequel la quantité comprend une superficie d'un crachat dans une image.

4. Système selon l'une quelconque des revendications 1 à 3, dans lequel une évolution de guérison comprend un stade de BPCO ou un événement d'aggravation prévu.

5. Système selon la revendication 4, dans lequel le système de commande est en outre configuré pour traiter la première image afin de produire une image prédite du crachat après un premier intervalle de temps.

6. Système selon la revendication 5, dans lequel le traitement de la première image comprend la modification d'une couleur d'un sous-ensemble de la partie des pixels.

7. Système selon la revendication 5, dans lequel la détermination de l'évolution de guérison comprend en outre la réception d'une première entrée provenant d'une interface utilisateur.

8. Système selon l'une quelconque des revendications 1 à 7, dans lequel la détermination de l'évolution de guérison comprend la détermination de savoir si le patient est proche d'une étape importante d'une évolution de BPCO.

9. Système selon la revendication 8, dans lequel le système de commande est en outre configuré pour afficher une image en stock associée à l'étape importante.

10. Système selon l'une quelconque des revendications 1 à 9, dans lequel la détermination de l'évolution de BPCO est basée sur un rythme de changement du premier ensemble de caractéristiques de crachat et du second ensemble de caractéristiques de crachat.

11. Système selon l'une quelconque des revendications 1 à 9, dans lequel la détermination de l'évolution de BPCO comprend en outre la réception d'une première entrée provenant d'une interface utilisateur.

12. Procédé de surveillance de BPCO, le procédé comprenant :
la réception, par un système de commande comprenant un ou plusieurs processeurs, d'une première image produite par une caméra avec un premier horodatage et d'une seconde image produite par la caméra avec un second horodatage ;
la comparaison des premier et second horodatages pour déterminer une période de temps entre les première et seconde images ;
le traitement des première et seconde images pour identifier une première partie de la première image et une seconde partie de la seconde image qui représentent un crachat ;
le traitement de la première partie pour identifier un premier ensemble de caractéristiques de crachat et le traitement de la seconde partie pour identifier un second ensemble de caractéristiques de crachat ;
le traitement des premier et second ensembles de caractéristiques de crachat et de la période de temps pour déterminer une évolution de BPCO ; et
l'affichage de l'évolution de BPCO sur l'écran.
